# EUROPEAN PATENT APPLICATION

(11) **EP 4 442 286 A1**
(43) Date of publication of application: **09.10.2024**
(21) Application number: 24168230.1
(22) Date of filing: 03.04.2024
(51) Int. Cl.: A61L 2/22, A61L 2/24, A61L 9/22, F24F 8/30, H05H 1/00, A61L 2/14

(54) **METHOD AND SYSTEM FOR THE SANITISATION OF SURFACES IN A CONFINED ENVIRONMENT**

(30) Priority: 03.04.2023 IT 202300006549
(71) Applicant: Amil Care Italia S.r.l., 21049 Tradate (VA) (IT); Longo, Matteo, 10064 Pinerolo (TO) (IT)
(72) Inventor: Longo, Matteo, 10064 Pinerolo (TO) (IT); Capobianco, Veronica, 21049 Tradate (VA) (IT)
(74) Representative: Fisauli, Beatrice A. M.

(57) **Abstract**

The present invention relates to a method for carrying out, tracing, monitoring and controlling an airborne disinfection treatment of surfaces of a confined environment, using a system of devices and a decontaminant.

## Description

The present invention relates to a method which uses a sanitizing technology by means of diffusion of a decontaminant. In particular, such a method uses a system of devices and said decontaminant.

In particular, it relates to a method for carrying out, tracing, and controlling a sanitizing process of surfaces, such as those delimiting a confined environment and of an object present within said confined environment, and, possibly, of air in such a confined environment.

Said method is performed by means of a sanitizing apparatus and, possibly, an air treatment device.

The confined environment, within which the method according to the present invention is carried out, can be any environment, typically it is a confined hospital environment, for example a clinic, a critical care room, etc. The confined environment can also be an industrial environment, or a public environment, such as an office, or a private environment, such as a domestic environment.

The environment to be sanitized generally contains at least one object, usually a plurality of objects. The objects contained in the confined hospital environment are, by way of example, medical devices, such as a bed, equipment, such as a defibrillator, an electrocardiograph, etc. It may be desired, possibly, to also sanitize at least one object present in the confined environment.

In the present description the terms sanitizing and disinfection are used interchangeably, unless otherwise specified.

The decontaminant is, preferably, a mixture formed by a chemical in a liquid medium, usually an aqueous medium. Said chemical product is the active substance having a sanitizing action.

In the field of airborne surface sanitizing of confined environments, the use of exothermic misting technologies paired with disinfectant chemical products is frequent. These exothermic misting technologies are proposed on the market in different use solutions.

There are devices which require a manual configuration by a professional operator and semi-automatic devices integrated with control and traceability systems which allow to document having carried out a disinfection treatment. More in general, in all the solutions proposed, it is a misting device consisting of an electric motor associated with a fan which sucks air from the confined environment and proportionally mixes, by means of a non-variable draft cannula, a mixture of air and misted decontaminant. As described for example in WO 2014/006577 A1, the traceability consists of the introduction of sensor systems for monitoring a disinfection activity by means of the automatic acquisition of the technical features of the confined environments and of the active substance dispersed in the air, reducing the manual intervention of the operator and documenting the activities carried out on a network platform.

These misting technologies, regardless of their level of innovation, have technological aspects which negatively influence the correct methodology of use of the active substance dispersed in the air within a confined environment, highlighting, for this reason, a limitation thereof both in use and in replicability. of action.

In order to ensure the correct execution of a sanitizing activity of surfaces by air which are contained within a confined environment, ensuring a conforming replicability with each intervention, it is essential that the misting technology easily adapts to a procedure and to an execution method which is proportional to the resistance of the microorganisms to be eradicated as a function of some environmental execution parameters.

The main environmental and non-environmental parameters to consider are the following:
- temperature and humidity of the air in the confined environment;
- air dispersion speed of the active substance and saturation speed of the confined environment;
- concentration of the active substance dispersed in the air within the confined environment, i.e., the concentration of the active substance in the air volume of the confined environment;
- contact time and action of the active substance with the surfaces and microorganisms contained in the confined environment;
- use of an active substance compliant with the chemical-physical features declared by the manufacturer; and
- possibility of interchanging the disinfecting active substance within the same operational use protocol.

The operating protocol establishes the procedure for carrying out the disinfectant treatment.

Knowledge of said environmental parameters relating to the confined environment to be sanitized is important for a correct sanitizing process of an environment.

Among the environmental parameters, the main physical parameters, or objective variables, to take into consideration are temperature and humidity. Said two variables act on the aqueous medium containing the decontaminant and on the mixture misted in the air of the confined environment. More in detail, a relevant parameter which such variables modify is the dew (or condensation) point of the mixture. Said variables can accelerate, or delay, reaching the dew point, thus anticipating or postponing it, respectively, without the operator being able to realize it. Dew point control is a critical action of any decontamination process, since a low concentration of an active substance could produce negative effects on the disinfection effectiveness, just as the accumulation of a concentrated active substance could have negative effects on materials, on the aeration time as well as on the effectiveness of a uniform disinfection.

Usually both the temperature and humidity within the confined environment to be disinfected undergo a variation. In fact, the combined use of decontaminants transformed from the liquid state to the semi-gaseous state by means of exothermic misting technologies produces, exponentially to the volume to be sanitized, an increase in the temperature of the confined environment and, at the same time, of the product dispersed in the air by the misting device.

The temperature increases due to the heat generated by the overheating of the electric perfusion fan which usually equips the sanitizing apparatus; due to its rotation, the fan raises the operating temperature and of the aspirated air which is subsequently introduced into the environment by the air treatment device.

The prolonged misting of the decontaminant within the confined environment also causes an increase in relative humidity, causing the onset of condensation on the colder surfaces.

The relevant execution parameters which influence the outcome of the sanitizing process include the air dispersion speed of the active substance and saturation speed of the confined environment. In particular, the misting speed is a parameter to take into account, especially if the sanitizing apparatus is used for overly large volumes. In most cases, due to the size and the high amount of active substance required, it involves the introduction, in the confined environment, of a high amount of mixture with the active substance, this causes high humidity in such an environment with consequent rapid reaching of the dew point of the active substance introduced in the confined environment, i.e., the active substance is converted into humidity already in the generation step and its degradation process occurs a few minutes after its dispersion in the confined environment. This aspect, if not appropriately monitored and managed, involves an immediate process of reducing the effectiveness of the active substance, preventing or completely excluding the achievement of the expected concentration.

A second important parameter is the concentration of the active substance dispersed in the air within the confined environment. The dispersion of the active substance in the confined environment until its minimum effective concentration is obtained, which must be mandatorily declared by the manufacturer in relation to the marketing authorization for the decontaminant used, is another fundamental aspect to monitor. The declared effective concentration of the active substance used within the decontaminant varies in proportion to the resistance of each individual microorganism.

All exothermic technologies which carry out the sanitizing treatment of surfaces in a confined environment are limited to introducing a known amount of active substance per single cubic metre of volume to be treated, regardless of the type of microorganism to be eradicated. Therefore, said relationship is usually not taken into account when sanitizing an environment. Ignoring such a relationship when carrying out the sanitizing treatment has negative consequences. In most cases, said neglect induces an overdosing of the concentration of the decontaminant dispersed in the air. The drawback which the dispersion of large amounts of active substance produces is that it progressively facilitates a resistance of the microorganism to the active substance itself. In the opposite case, i.e., an under-estimated concentration of active substance, the hypothesized qualitative result will not be achieved.

Other relevant parameters are the contact time and action of the active substance with the surfaces and with the microorganisms contained in the confined environment.

The manufacturer of the decontaminant is obliged, on the basis of the technical and scientific documentation in his possession, to declare the methods of use of the chemical product, specifying what concentration must be used and the contact time to envisage for the purposes of obtaining the declared action result. Each microorganism has a different level of resistance when brought into contact with a known concentration of active substance.

Carrying out a disinfection treatment requires the use of a product which complies with the chemical-physical features declared by the manufacturer. The chemical-physical features declared by the manufacturer can be guaranteed only and exclusively until the guarantee seal placed on the original packaging is removed. Starting from the removal of the guarantee seal, the active substance contained in the decontaminant, even if kept in its original packaging, may undergo a more or less accelerated degradation or chemical-physical alteration. The alteration can be a consequence attributable to multiple factors, including, for example, the storage temperature, exposure to light, heat sources, contact with the oxygen present in the environment.

Lastly, it is scientifically documented that the prolonged use of the same active substance, to counteract the emergence of infection within a confined environment, can create microbial resistance in the long term.

In addition, possible errors in the traceability of the disinfection carried out very often manually by operators can lead to repercussions also on the health of the operator and on the safety of the activities envisaged within the confined environment following sanitizing procedures.

The Applicant has now found a surface sanitizing treatment method by means of which the aforementioned drawbacks caused by the known methods are overcome.

Furthermore, the present invention proposes a method which allows the aforesaid parameters to be simultaneously acquired and verified in the start-up step and while carrying out a sanitizing procedure, including their chronological validation in the monitoring procedure and conformity of use, improving the reliability of the disinfection technologies.

An advantage of the present invention is that of providing a method which envisages a procedure for verifying, monitoring and tracing the sanitizing throughout the sanitizing treatment.

In particular, the method of the present invention allows to document that the sanitizing occurred according to a predefined operating protocol corresponding to a specific EN standard distinct for bacterial strain.

For example, with such a method the contact time of the misted disinfectant and the surfaces and objects present within the confined environment is controlled, at an established concentration, and, possibly, between an ionized gas and the air contained in a confined environment, and it is verified that such a contact time corresponds to that envisaged by the reference operating protocol.

As part of a further verifying, monitoring and tracing step, a further advantage of the method of the present invention, with respect to the technologies currently on the market, is that it allows to have evidence of the contact time with the surfaces and with the microorganisms, using a known concentration of active substance, for the time sufficient to certify the result of efficacy and action.

Furthermore, the method according to the present invention guarantees that the times for restoring the minimum safety conditions, according to current legislation, to be able to stay in the disinfected confined environment have been appropriately verified and chronologically documented.

A further advantage of the present method is that it can provide the guarantee of being able to use a product which conforms to the chemical-physical features declared by the manufacturer, i.e., that it has not undergone a degradation or chemical-physical alteration. This is because the decontaminant inside the pack is consumed entirely during the same treatment cycle.

A further advantage of the present method is that it gives the possibility of interchanging the active disinfecting ingredient within the same operational use protocol.

An object of the present invention is to provide a method for continuously sanitizing surfaces in a confined environment, in particular a method for carrying out, tracing and controlling a disinfection treatment of surfaces, and possibly of air, in a confined environment.

A further object of the present invention is to create a sanitizing method by means of a decontaminant possibly combined with an air treatment device, which can configure its operating cycle proportionally to the microbiological effectiveness result to be achieved, to the volume to be treated and to the environmental parameters detected within the confined environment in which its use is envisaged.

An object of the present invention is to create a sanitizing method which takes into account at least one and, preferably, two or more environmental parameters, in particular those relating to pollutants (for example PM 1-2.5-10, TVOC, CO₂ and Environmental Quality Index) and those of the microclimatic parameters, particularly temperature and humidity, of the confined environment in which its use is envisaged.

Another object of the present invention is to provide the sanitizing apparatus with a universal quick coupling system for the interchangeable use of decontaminant contained in a single-use and single-dose pack. The use of a pack of such type allows the chemical and physical features of the decontaminant to be kept unaltered, and thus its decontaminating and/or disinfecting properties, to be used in a confined environment up to its sole and complete use.

Another object of the present invention is to detect the concentration of an airborne active substance in the form of mist in a confined environment, allowing the precise monitoring of the contact and action time of the decontaminant with the surfaces, objects and any undesired microorganisms contained therein, necessary to determine the conclusion of the sanitizing activity.

Another object of the present invention is to chronologically document the steps for carrying out the sanitizing cycle.

Another possible object of the present invention is to detect, if present, the concentration in gaseous form of an ionized gas dispersed in the air in a confined environment, allowing the precise monitoring of the contact time and action of the decontaminating gas with the air and any undesired microorganisms contained therein in conformity with the safety parameters established by the automatic configuration system and for the time necessary to establish the conclusion of the sanitizing activity.

These and other objects and advantages, which will be better understood below, are achieved according to the present invention by a method for carrying out, tracing and controlling an airborne sanitizing treatment of surfaces of a confined environment and of at least one object present therein having the features defined in the attached claims.

In detail, said method for carrying out, tracing and controlling a disinfection treatment is carried out by means of the use of a system of devices comprising at least:
- a sanitizing apparatus provided with at least dispensing means of a liquid decontaminant contained in an interchangeable pack,
- a control unit comprising at least:
   a) first sensor means for detecting the concentration of said decontaminant in the confined environment,
   b) a combined sensor, and
   c) control means and processing means, said control and processing means being connected to said first sensor means.

Said control unit is configured to contain identification information of the confined environment.

In particular, said combined sensor is designated to detect at least one environmental parameter relating to at least one piece of data selected from microclimate data and data concerning the presence of at least one pollutant.

Said decontaminant is released in the form of mist. The dispensing of said decontaminant is controlled as a function of at least one of the pieces of data detected by said first sensor means. The dispensing is conducted according to a predetermined operating protocol for carrying out the treatment.

The method according to the present invention comprises the steps of:
a) associating the control unit with the confined environment;
b) arranging said object in the confined environment;
c) arranging said sanitizing apparatus in the confined environment;
d) acquiring information concerning at least said environmental parameter by means of said combined sensor;
e) processing, with said control means and processing means, said information acquired by means of said combined sensor, producing said operating protocol for carrying out the disinfection treatment, the disinfection mode being defined in said protocol;
f) transferring data relating to the disinfection protocol to the sanitizing apparatus to initiate the disinfection treatment of the confined environment and of the at least one object;
g) inserting the pack into the sanitizing apparatus;
h) dispersing into the air said decontaminant according to a concentration and for a dispensing time calculated by the control and processing means based on the information contained in said operating protocol; and
i) making available certification information, according to the concentration of decontaminant detected, to certify that disinfection of the surfaces of the confined environment and of the at least one object present therein has taken place during the step of dispensing the decontaminant.

Said certification information made available is related to the concentration of decontaminant detected as a function, generally, for example, of the action time required to eliminate the bacterial strains established during the configuration of the operating protocol.

The disinfection treatment conducted according to the method of the present invention uses said chemical product, which is usually in liquid form or in a liquid medium. Said decontaminant used is in a packaged form in a container, preferably disposable and single-dose. Such a pack is of the interchangeable type.

The control unit, associated with the confined environment, is provided with detection sensor means. The control unit is combined with the environment to be sanified, therefore la unit memorizes, with reference to the environment to be sanified, the relevant data relating to that environment.

Such detection sensors allow to detect the presence of, for example, pollutants, decontaminants and values relating to environmental microclimatic parameters.

The control unit is configured to contain identification information useful for carrying out the sanitizing, for example relating to the specific confined environment to be treated, such as its volume, the type of microbiological pollution (i.e., bacteria, fungi, viruses, spores and mycobacteria) to be eradicated in the confined environment to be treated, the optimal dosage range of decontaminant to be dispensed (minimum and maximum) as a function of various parameters, including, for example, the microclimatic parameters within the confined environment, the concentration of decontaminant dispersed in the air, the contact time and action of the active substances, the exposure limits and treatment frequency, the objects present in the confined environment and the possible concentration of gases dispersed in the air.

In relation to the volume of the confined environment, this is important for knowing the volume of air to be treated. The volume of air in the confined space can be considered and calculated with reference only to the size of the confined space. Such a calculation produces a value corresponding to the maximum possible volume of air in the confined environment (defined herein as "maximum possible volume of air").

Alternatively, the calculation of the air volume is performed also taking into account the presence of at least one object to be sanitized or even of any further object present therein. Such an object subtracts air present within the confined environment, therefore if it is considered in the calculation of the volume of air, the volume of air is smaller with respect to the aforementioned possible volume of air, an effective volume of air will be obtained.

With reference to the volume of air in the confined environment, the system can therefore be set, by choice, whether to calculate the maximum possible volume of air or the actual volume of air and, consequently, which of the two volumes to consider as identification information.

Said identification information is essential to establish an operating protocol for the treatment of the individual confined environment.

By means of the combined sensor, information relating to the microclimatic parameters, mainly temperature and humidity, present within the confined environment is acquired. Therefore, the temperature and humidity of the air in said environment are usually measured, as is the dew point.

The sanitizing apparatus used in the method of the present invention is known per se, as for its specific construction and operating characteristics of the mechanical parts, for example it is described in WO 2014/006577 A1 and in IT 102012902055929, therefore it will not be described here in a detailed manner.

In short, the sanitizing apparatus can comprise a misting device connected to dispensing means for the diffusion of the decontaminant by air, a fan and an electric motor associated with the fan, a feeding and suction cannula adapted to withdraw the decontaminant contained in a pack and an interchangeable coupling system of decontaminant packs.

In the sanitizing apparatus, the decontaminant and the air coming from the confined environment are mixed proportionally in the proportion established by the operating protocol. The decontaminant is introduced into the air to be disinfected, and aspirated from said confined environment, in a proportion typically comprised between 0.1 ml to 20 ml per cubic metre of air. The decontaminant is introduced into the air to be disinfected in an amount which generally depends on the humidity and the temperature which such air has. Generally the humidity is from 5 to 75% and the temperature between 5 and 60°C. Indicatively, the decontaminant is introduced into the air in amounts of 3 ml per cubic metre of air in the case of air having a humidity of 40% and a temperature of 25°C.

The air/decontaminant mixture is misted by the misting device. Said dispersion in the air typically occurs by means of the Venturi effect.

The fan draws, by Venturi effect, the decontaminant from the pack through the cannula, aspirates air from the confined environment, proportionally mixes the decontaminant with the sucked air by means of a flow regulation valve and emits a mixture of air and misted decontaminant into the environment through a diffuser. The dimensions of the particles emitted in the confined environment are calibrated by the aforesaid flow regulation valve located between the suction cannula and the diffuser.

The sanitizing apparatus can comprise, among other things, means for recognizing the interchangeable pack. In such a case, the method according to the present invention further comprises, preferably, enabling the use of the disposable and single-dose pack, containing the decontaminant inserted on board the sanitizing apparatus, by means of said means for recognizing the decontaminant. Said enabling occurs in step (g) and following the insertion of the interchangeable pack in the sanitizing apparatus.

Preferably, to improve the efficiency of the method of the present invention, such a method also comprises the steps of:
α) detecting the concentration of the decontaminant dispersed in the air in the confined environment;
β) calibrating the operation of the misting device, modulating the amount and input of decontaminant dispersed in the air inside the confined environment in conformity with the specifications indicated in the operating protocol.

Said steps (α) and (β) are performed during the disinfection treatment to regulate the concentration of decontaminant.

As mentioned above, the confined environment usually contains one or a plurality of objects.

It may be desired to specifically sanitize at least one of the objects present in the confined environment. In such a case, according to a preferred variant of the method in accordance with the present invention, each object which is desired to be sanitized (hereinafter defined as "target object") is provided with a unique identification device. Such an identification device contains identification information of the target object, as explained below. The object identification device operates within an identification system.

According to such an embodiment of said method, the system of devices, in particular the control unit, also comprises recognition means. The latter are configured to acquire the identification information of the target object placed inside the confined environment sent by the identification device. Thereby the target object communicates with the control unit.

Therefore, in such an embodiment, the method according to the present invention also comprises a further step (γ) of sending information from the identification device associated with at least one target object to the recognition means comprised in the control unit.

Once said identification information has been acquired, such information is processed by the control means and by the processing means in the aforementioned step (e), together with the other information acquired in order to produce said operating protocol for carrying out the disinfection treatment.

Therefore, said step (γ) is conducted before the aforesaid step (e), typically after step (c), generally before or after or together with step (d).

According to a further embodiment, the method of the present invention also comprises an air decontamination treatment.

Such a decontamination or sanitizing of the air is conducted with a specific air treatment device. Such a device can be, for example, a cold plasma ionization device. Preferably such a device is integrated in the misting device.

The cold plasma ionization technique is well known. Briefly, such a technique uses the ionization of air, transforming the latter into ionized gas. Such a treatment involves a system for recirculating the volume of air contained within the confined environment. In such a case, the method of the present invention therefore, possibly and further, also comprises the following steps:
δ) arranging an air treatment device, dispenser of cold plasma gas, inside a confined environment;
ε) transferring the operating specifications and the amount of plasma gas to disperse into the air of the environment to the air treatment device;
η) detecting the concentration of plasma gas dispersed in the air by the air treatment device in the confined environment;
θ) calibrating the operation of the air treatment device modulating the amount of plasma gas dispersed in the air inside the confined environment in conformity with the specifications indicated in the operating protocol; and
κ) providing certification information relating to the concentration of plasma gas detected as a function of the exposure time required to eliminate the bacterial strains established during the configuration of the operating protocol.

Said step (δ) is conducted before the aforesaid step (e), typically before or after any step from (a) to (c). The subsequent steps (ε), (η) and (θ) are generally carried out after said step (d). Said step (κ) is carried out, usually but not necessarily, after step (i).

The air treatment device comprises a memory and control card which controls the operation of an ionizing tube, the intensity of which is regulated, for example, by means of a potentiometer. The plasma gas is conveyed into the confined environment by an air recirculation fan.

The detection of environmental parameters, in particular temperature and humidity, in relation to the concentration of the decontaminant mist and, possibly, to the action and contact time of the plasma gas dispersed in the air serves to guarantee the expected qualitative result, i.e., sanitizing, disinfection, high disinfection or sterilization of the confined environment.

According to such an embodiment, the sanitizing apparatus and the air treatment device can be independent of each other or, preferably, can be integrated, forming a single piece of equipment positioned, for example, within the confined environment.

The control unit can be located permanently or removably in the confined environment.

The control unit is outside or, preferably, inside the confined space.

The control unit can be stand-alone or integrated in another device comprised in the system of devices, for example, in the sanitizing apparatus or, if present, in the air treatment device.

The control unit usually comprises the following:
i. first sensor means, adapted to detect the concentration of decontaminant dispensed in the confined environment,
ii. a combined sensor for detecting environmental microclimatic parameters in the confined environment,
iii. control means,
iv. processing means, and
v. memory means, and possibly
vi. second sensor means, adapted to detect gas.

Through the control and processing means, the operating activities of said technologies and the use of said products are coordinated and documented chronologically according to the execution methodology described in detail below.

As explained above, the control unit receives, processes and then makes the information available which uniquely identifies the confined environment to be treated.

In particular, the control unit receives and processes information relating to the architecture of the confined environment, such as the volume, plant equipment or other, and those of the environmental parameters.

In addition, the control unit can contain information relating to the specific infectious emergence features of the activity carried out within the confined environment, such as contamination by bacteria, fungi, viruses, spores or mycobacteria.

Furthermore, the control unit can contain information on one or more of the following aspects including the periodicity of carrying out the decontamination treatment, the optimal environmental microclimatic parameters for carrying out an air treatment activity and a surface disinfection activity, the active substance and the recommended concentration of the decontaminant to be used, the contact and action time with the surfaces and objects to be respected or on the parameters for restoring the minimum safety conditions relating to carrying out the decontamination treatment performed in the confined environment.

The decontaminant is contained in a pack, preferably of the disposable type and, possibly, also single-dose.

The decontaminant is preferably associated with an identification device. The latter is typically located on the pack of the decontaminant.

As previously mentioned, the confined environment usually comprises objects, which can be fixed in the confined environment and/or movable, therefore transportable from one confined environment to another confined environment.

Said recognition means can comprise a memory card and means for identifying the at least one target object, such as devices configured to communicate an RFID circuit or optical or equivalent reading devices, configured to read a barcode or a QR code, said RFID circuit or barcodes or other types of encoded data being the identification devices applied to the objects in the confined environment.

According to a variant of the invention, one or more of the object identification devices can comprise a low-power wireless communication module (for example Bluetooth Low Energy, BLE), i.e., a unique geo-locatable identification code. As is known, each identification system (which, for example, can be based on BLE technology) is distinguished by a unique identification serial number, i.e., an identification code. Such a code can be associated with an object.

The identification device is, for example, or can comprise a label.

In a preferred embodiment variant, the recognition means can be configured to acquire identification information of the decontaminant to be disseminated in the confined environment by the sanitizing apparatus.

Such information is contained in an identification device of the decontaminant.

Said identification device of the decontaminant contains identification information relating to the decontaminant to be dispensed, such as the type of active substance, production date, expiry date, concentration, etc.

The identification device of the decontaminant can also comprise a label within which an RFID circuit, a bar code, or a QR code can be present.

Said identification device of the decontaminant can be located on its pack.

As previously mentioned, the control means and the processing means are configured to perform the calculation of the concentration and dispensing time of said decontaminant. In addition, the control means and the processing means can be configured to store and process further information as well, which is, depending on the case, for example, the identification information of the decontaminant and the identification information of the objects contained in the confined environment, acquired by the recognition means, the information on the concentration of decontaminant dispersed in the air in the confined environment acquired by the detection sensor.

Depending on the embodiment of the method of the present invention, the control means and the processing means can be configured to supervise the operation and/or coordinate the activity of the valve regulating the flow of the decontaminant dispensed by the nebulizing device. In particular, such control and processing means can also supervise the start-up, operating and shut-down activities of all the devices associated therewith and/or control the activity of the decontaminant diffuser nozzle and of the air treatment device.

The detection of environmental parameters, generally temperature and humidity, acquired by the combined sensor, can be monitored periodically or continuously during the dispensing of said decontaminant.

Likewise, the detection of environmental parameters, generally temperature and humidity, acquired by the combined sensor, can be monitored periodically or continuously during the dispensing of said plasma gas.

The features and advantages of the invention will emerge from the detailed description of some of its embodiment examples made with reference to the attached drawings, given by way of indicative and non-limiting purposes, in which:
- figure 1 is a perspective view of a confined environment with a system of devices used in a method according to an embodiment of the present invention;

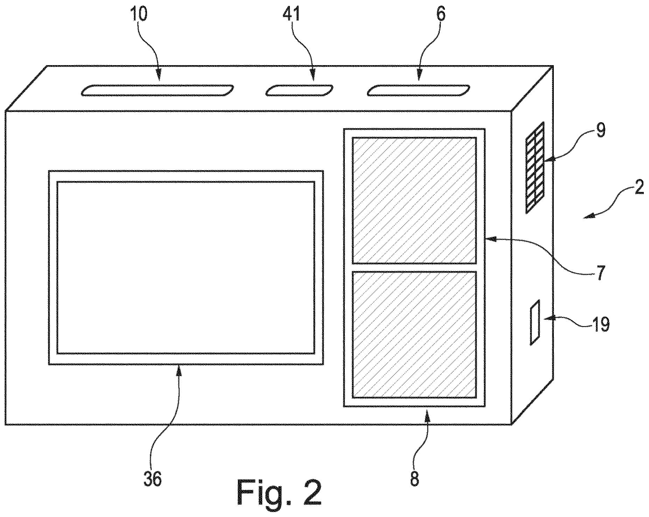

- figure 2 is a perspective view of a control unit comprised in the system of devices used in a method according to an embodiment of the present invention;
- figure 3A is a detail of a side view of a decontaminant misting device according to an embodiment;
- figure 3B is a detail of a front view of a decontaminant misting device according to an embodiment;
- figure 4A is a detail of a side view of a male universal adapter for packs according to an embodiment of the present invention;
- figure 4B is a detail of a bottom view of a male universal cap for packs according to an embodiment of the present invention;
- figure 4C is a detail of a side view of a female fixing system of the universal male cap according to an embodiment of the present invention;
- figure 4D is a detail of a front view of a female fixing system of the universal male cap according to an embodiment of the present invention;
- figure 5 is a perspective view of a system for treating air by means of plasma gas according to an embodiment of the present invention;
- figure 6 is a front view of an identification device of a pack of a decontaminant;
- figure 7 depicts two graphs automatically generated by the control unit;
- figure 8 depicts a graph concerning the configuration of a confined environment and an operating protocol configured in the start-up step of the treatment cycle according to an embodiment of the method of the present invention;
- figure 9A is a perspective view of recognition means, according to a second embodiment of the present invention;
- figures 9B, 9C are perspective views of objects provided with object identification devices, according to an embodiment of the present invention;
- figure 10 depicts a graph concerning the configuration procedure of an execution cycle guideline according to an operational protocol.

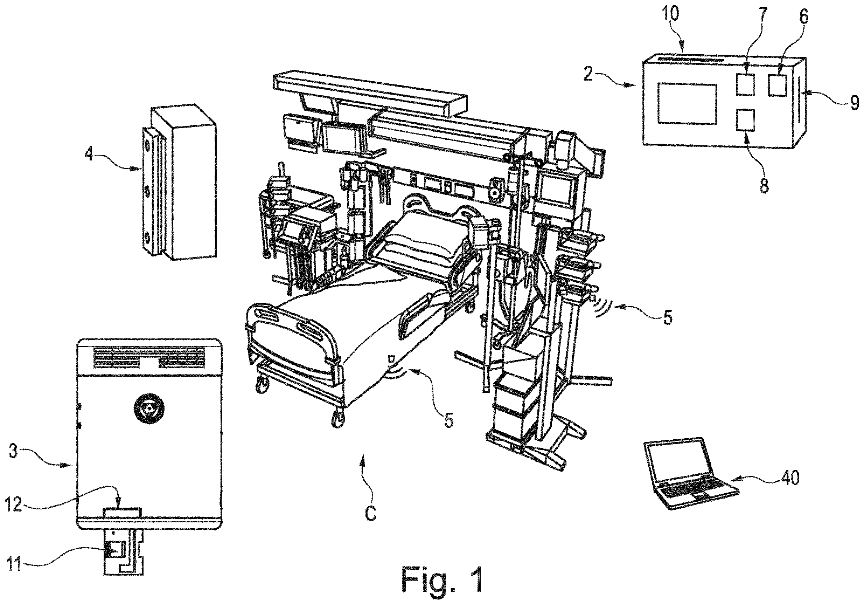

Referring initially to figure 1, a preferred embodiment will first be described of a method for carrying out, tracing, monitoring and controlling the disinfection of surfaces of a confined environment C, as well as of objects contained therein and of an air decontamination treatment activity.

The confined environment C is illustrated in such figure 1, depicting a hospital environment containing a plurality of objects, with at least one of which a unique identification device 5 is associated. In the example illustrated in figure 1, the objects contained in the confined environment C and associated with an identification device 5 are a bed and medical equipment.

A system of devices 1 is positioned in the confined environment C, comprising a sanitizing apparatus 3, a control unit 2, a plurality of objects, identification devices 5, associated with said objects and a decontamination and air recirculation treatment device 4.

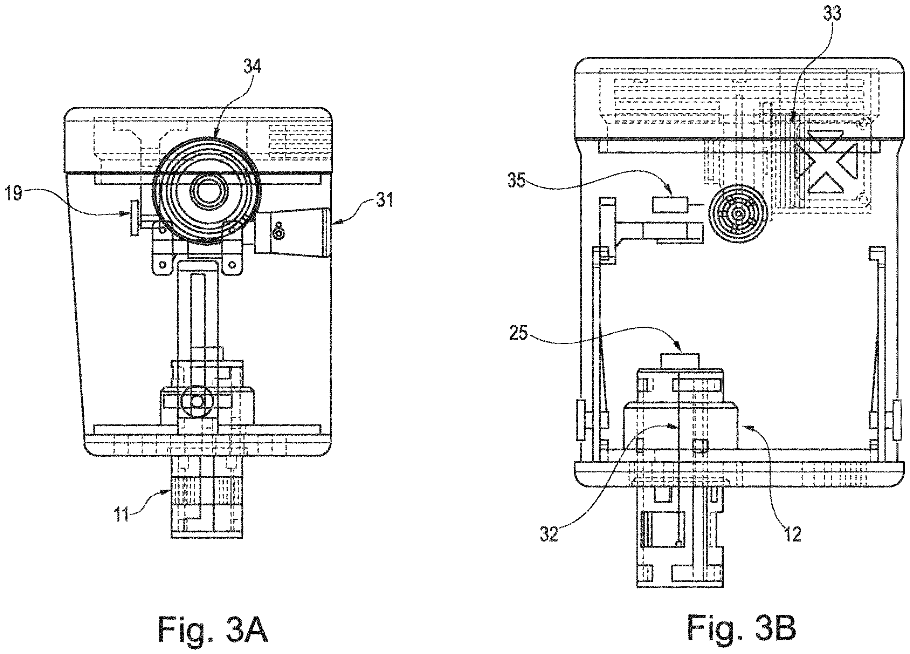

In such a method, a single-dose, disposable, interchangeable pack 25 containing a decontaminant 11 is used (fig. 3B).

An identification device 26 is located on the pack 25, which contains identification information of the decontaminant 11.

As illustrated in figure 3A, the sanitizing apparatus 3 is provided, among other things, with a diffuser nozzle 31 for the dispersion in the air of the decontaminant 11 and with recognition means 12 of the pack 25. The dispersion in the air of the decontaminant 11 occurs by means of Venturi effect, as explained in detail below.

The control unit 2 is provided with a display 36, recognition means 6, first sensor means 7 for detecting decontaminants 11, a combined sensor 41 for detecting environmental microclimatic parameters, control means 9, processing means 10, a memory card 19 and, possibly, second sensor means 8 for detecting gases dispersed in the air in the confined environment (C).

Said first detection sensor means 7 are configured to detect the concentration of the decontaminant 11 dispersed in the air by the diffuser nozzle 31 of the sanitizing apparatus 3 within the confined environment C.

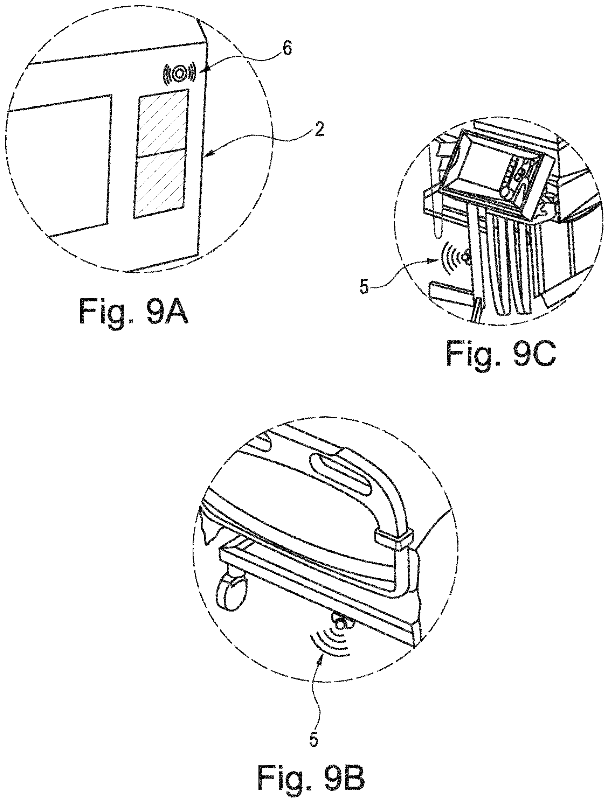

As visible in each of figures 9B and 9C, the identification device 5 associated with the objects is illustrated. The device 5 contains and makes identification information of the object with which it is associated available.

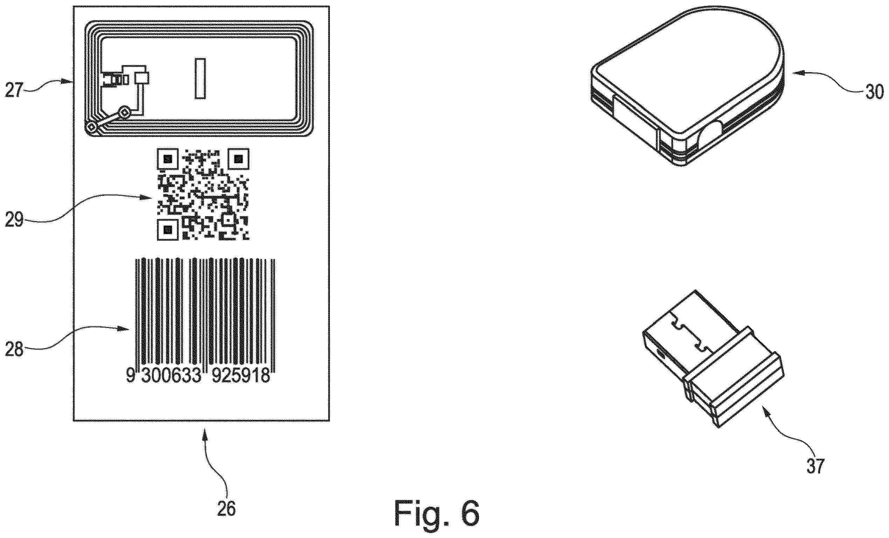

As illustrated in figure 6, the identification device 26 comprises a label within which there is an RFID circuit 27, preferably rewritable, a barcode 28 and a QR identification code 29.

As illustrated in figures 3A and 3B, the sanitizing apparatus 3 also comprises an interchangeable coupling system (described later in detail) of the pack 25 of the decontaminant 11, a feeding and suction cannula 32 adapted to withdraw the decontaminant 11 within the pack 25, a fan 34, an electric motor 33 associated with the fan 34, and a flow regulation valve 35.

The fan 34 draws, by Venturi effect, the decontaminant 11 from the pack 25 through the suction cannula 32. The decontaminant and the aspirated air mix, forming a mixture, proportionally by means of the flow regulation valve 35.

The mixture of decontaminant 11 and aspirated air are emitted in the confined space C through the diffuser nozzle 31 in mist form. The dimensions of the particles emitted in the misted mixture are calibrated by the flow regulation valve 35 placed between the suction cannula 32 and the diffuser nozzle 31.

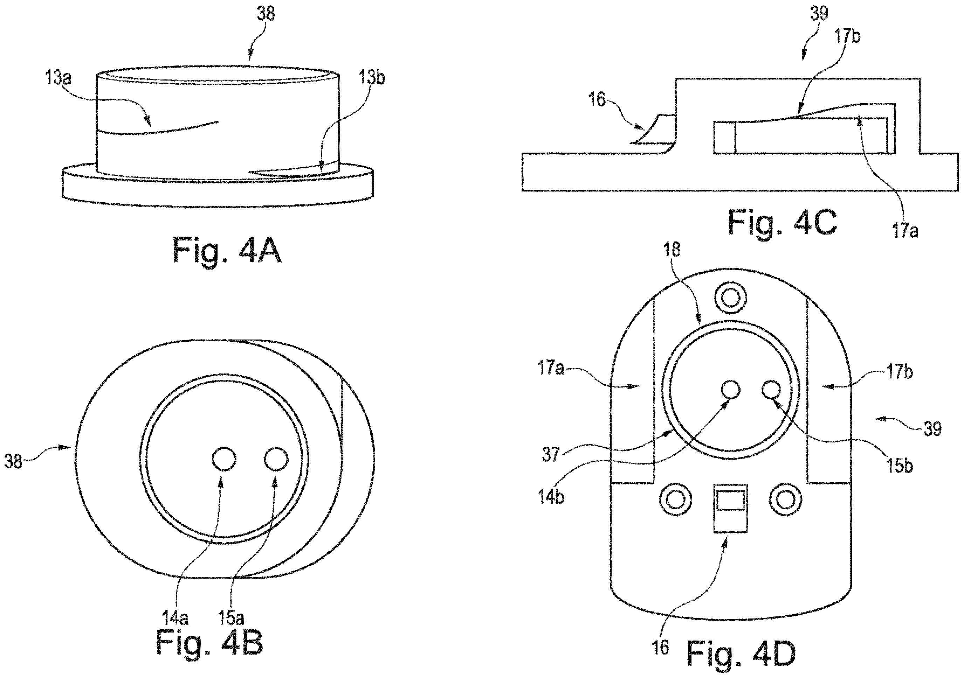

The sanitizing apparatus 3 is also provided with an insertion system of the pack 25. Such a system comprises a universal interchangeable male adapter 38 (figures 4A and 4B) and a female adapter 39 (figures 4C and 4D).

In detail, the male adapter 38 has a hole 14a for drawing the decontaminant, an air passage hole 15a and flaps 13a and 13b (figures 4A and 4B).

The female adapter 39 has a coupling stroke stop 16 and two fixing guides 17a, 17b for the mechanical seal and a seat 37 for the insertion of a gasket 18 with hydraulic seal 37 (figures 4C and 4D).

The male adapter 38 is screwed onto the pack 25 and coupled to the female adapter 39, by means of the fixing guides 17a, 17b which cooperate with the flaps 13a and 13b, respectively. The correct position of the male adapter 38 in the female adapter 39 is defined by the coupling stroke stop 16, visible in figure 4D which shows a detail of a front view of a fixing system present on the female adapter 39 to receive the male adapter 38.

In particular, the fixing guides 17b, 17b are shaped to allow a 90 degree rotation of the male adapter 38 in said guides 17a and 17b; thereby, total adherence of the male adapter 38 to the female adapter 39 is obtained. Furthermore, such a rotation allows the alignment of the passage holes of the decontaminant and the air, in particular of the passage hole 14a of the decontaminant 11 and of the air passage hole 15a present on the male adapter 38 with the corresponding passage hole 14b of the decontaminant 11 and air passage hole 15b present on the female adapter 39.

The hydraulic seal between the male adapter 38 and the female adapter 39 is guaranteed by the gasket 37, placed on the female support 39, which, due to the 90 degree rotation necessary to anchor the male adapter 38 to the female adapter 39, is pressed between said parts.

The recognition means 6 (figure 9) integrated in the control unit 2 can be configured to acquire the identification information contained in the identification devices 5 of the objects located within the confined environment C (figure 9A, 9B) associated with such objects. Embodiments can envisage that the recognition means 6 can modulate the size of the object search radius as a function of the volumetric dimension of the confined environment C.

The decontaminant is thus dispensed in the form of particles with dimensions comprised between 2 and 10 microns, preferably 2 microns in the case of ultra-fine mist, 5 microns in the case of dry mist, 7 microns in the case of mist and 10 microns in the case of fine spraying. Such a device prevents the deposit of the decontaminant on the surfaces and the creation of humidity.

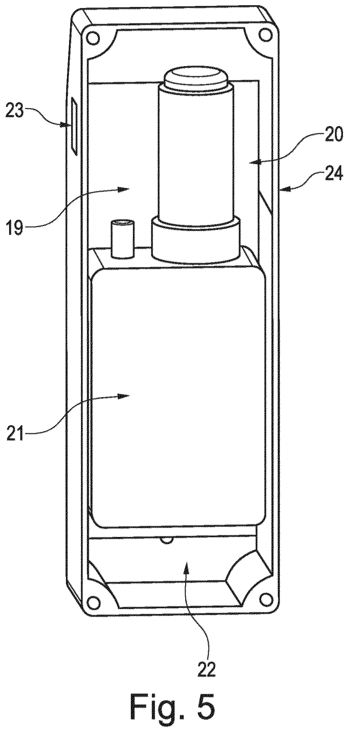

According to a preferred variant of the method of the present invention, the method also comprises an air treatment step which is performed with a treatment device 4, illustrated in figure 5. The air treatment device 4 comprises an ionizing tube 20, a potentiometer 22 to control the power of the ionizing tube 20, a fan 23, adapted to recirculate the air in the confined environment C, a grille 24 for expelling the disinfected air, a memory card 19 and control means 21. The latter control the operation of the air treatment device 4.

In such a variant, the air treatment device 4 is integrated in the surface sanitizing apparatus 3.

In a further embodiment, one or more of the components of the control unit 2, including the recognition means 6, the first detection sensor means 7 for detecting the concentration of decontaminant 11, the second detection sensor means 8 for detecting gas and the control and processing means 9, 10, can be mounted on the sanitizing apparatus 3.

As previously mentioned, the control means 9 and processing means 10 are configured to make certification information available, in order to certify the successful disinfection (or not) of the surfaces and any objects contained in the confined environment C.

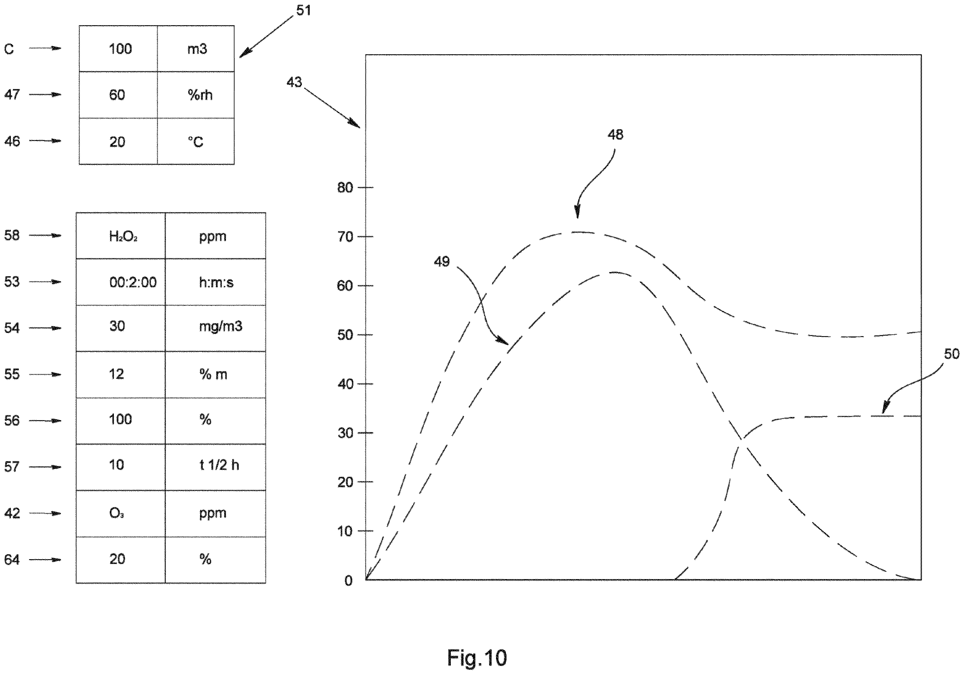

More in particular, according to the method of the present invention, said control means 9 and processing means 10 are configured to arrange, as a function of the microclimatic parameters including those of temperature 46 and humidity 47 detected within the confined environment C, an optimal guideline for carrying out the treatment cycle summarized in an execution graph 43 (figure 10) showing a relative humidity curve 48, a relative saturation curve 49 of an active substance 58 of a decontaminant 11 and a relative saturation curve of a plasma 42 gas dispersed in the air.

Said control means 9 and processing means 10 are further configured to compare the effective concentration values 44 of decontaminant detected by the first sensor means 7 and the effective concentration values 45 of plasma gas 42 dispersed in the air detected by the second sensor means 8, during the treatment cycle, with said relative value curves, to establish whether or not the disinfection has been successful.

According to a further variant, the system of devices 1 can be associated, in a known manner, with a central server 40 (figure 1) adapted to coordinate the activity of the various devices of the system.

In such a variant, the central server 40 can be arranged to receive information relating to the disinfection process of the surfaces of the confined environment C and of any objects contained therein, and of any air treatment, and in particular the certification information made available by the control means 9 and processing means 10 on the conformity or non-conformity of the disinfection of the surfaces of the confined environment C and of any objects contained therein and of any air treatment during the dispensing step of the decontaminant 11 and, possibly, that of dispersion of the ionized gas in the air.

The certification information can be transmitted to the server 40 by means of the control unit 2. Such transmission can occur with any telecommunications technology, for example wirelessly, such as Bluetooth, SMS, or GPRS, or via cable. Alternatively, such certification information can be loaded by the control unit 2 onto a removable storage medium, for example a USB key, and then provided to the server 40.

The data acquired by the central server 40 can be processed by the server 40 itself so as to highlight anomalies with respect to the expected objective and certify the disinfection activities carried out and their relative chronology, in accordance with a pre-established self-control plan (which relates to the planning of the activities envisaged within the confined environment) and with the creation of a "technical operating dossier" defined for each disinfected confined environment C and, possibly, for each individual disinfected object 5.

The steps of a preferred embodiment of a method for carrying out, tracing and controlling an airborne disinfection treatment of the surfaces of a confined environment C and of the objects contained therein and of air decontamination will now be described.

Preliminarily, the control unit 2 is configured to contain identification information of the confined environment C to be sanitized, its volume and the critical issues of infectious emergence catalogued in order of resistance.

Then a plurality of objects are arranged in the confined environment C, each provided with an associated identification device 5 which contains identification information of the object and the following devices:
- the control unit 2, previously configured;
- the sanitizing apparatus 3, adapted to dispense the decontaminant 11 by air for the treatment of the surfaces and objects; and
- the device 4 for air treatment and recirculation by means of plasma gas.

Then the method proceeds with the acquisition of:
- information on polluting agents, temperature 46 and humidity 47 present within the confined environment C by means of the combined detection sensor 41; and
- identification information of the objects by means of the recognition means 6 and the identification device 5 associated with each object.

An operating protocol for disinfecting surfaces, objects and treatment of the volume of air contained in the confined environment C is arranged through processing means 10.

The operating protocol is customized in real time on the environmental parameters acquired by the combined detection sensor 41 and, proportionally, on the classification of infectious emergence (bacteria 59, fungi 60, virus 61, spores 62 and mycobacteria 63) and other parameters, such as the active substance 58 to be used, the dimension of the particles 56 to be introduced in the confined environment C, the dispensing speed 54 of the decontaminant 11, the starting concentration 55 in the liquid state of the decontaminant 11 to be dispersed in the air in a confined environment C, the overall duration of the treatment 53 and the contact and action time 65 of the decontaminant 11 and the gas, recovery time 57, intervention frequency, the start time of the dispensing cycle, the start-up and shut-down mode of the devices 3 and 4.

Said recovery time is the time required for the safety parameters established by the safety standards to be reached. Such parameters are indicated in the safety data sheets of the active substance.

The control unit 2 transfers, by means of the control means 9, the operating commands to the sanitizing apparatus 3 and to the air treatment device 4.

The control unit 2 coordinates the switching on, operation and switching off of the sanitizing apparatus 3 by means of the combined sensor 41 and the first sensor means 7.

Initially in the sanitizing apparatus 3, the recognition means 12 of the decontaminant 11 are activated by the control unit 2 to enable the insertion of the disposable and single-dose pack 25 containing the suitable decontaminant 11. Then the sanitizing apparatus 3 carries out a conformity check of the single-dose and disposable pack 25 inserted on board and activates the operation of the electric motor 33 and the fan 34 which, by means of the diffuser nozzle 31, disperse the decontaminant 11 in the air in the confined environment C.

The control means 9 and processing means 10 record all the steps of the procedure of carrying out, tracing and controlling a sanitizing treatment, including the times of achievement of each individual objective indicated in the operating protocol relating to: bactericidal 59, fungicidal 60, virucidal 61, sporicidal 62 and mycobactericidal 63 effectiveness, concentration 55, contact time and restoration of minimum safety conditions.

As previously specified, the control unit 2 controls the switching on and off activities of the sanitizing apparatus 3. Such switching on and off activities are carried out, if necessary, even several times during the sanitizing treatment cycle, to maintain the concentration level of the decontaminant 11 for the contact time necessary to determine the indicated effectiveness in the operating protocol.

The achievement of the effectiveness is determined on the basis of the active substance 58 of the decontaminant 11 which is dispensed relative to the parameters established by the processing means 10 with respect to the temperature 46 and humidity 47 values acquired by the combined detection sensor 41 in the volume of air contained in the confined environment C.

Once the disinfecting activity of the surfaces and objects has been completed by means of the use of the sanitizing apparatus 3, if desired, the treatment activity of the air contained in the volume of the confined environment C is proceeded with, by means of activating the air treatment device 4.

In an alternative embodiment of the method according to the present invention, the air treatment activity can be carried out before the surface and object disinfection activity.

The control unit 2 coordinates the switching on and off of the air treatment device 4 by means of the combined sensor 41 and the second sensor means 8.

The control means 9 and processing means 10 are arranged to record all the steps of the execution procedure including those of achieving each individual objective indicated in the operating protocol relating to: bactericidal 59, fungicidal 60, virucidal 61, sporicidal 62 and mycobactericidal 63 effectiveness, concentration 64 of plasma gas 42, contact and action time 65 and the restoration time 57 of the minimum safety conditions.

In detail, the control unit 2 coordinates the switching on and off of the fan 23, placed on the air treatment device 4, acting on the potentiometer 22 which controls the power of the ionizing tube 20, to maintain the effective concentration level 45 above the relative concentration curve 50 of the plasma gas 42 for the contact time with the air necessary to determine the effectiveness indicated in the operating protocol and in the procedure for configuring its concentration 64. Said switching on and off can also occur several times during the treatment cycle, depending on need.

Preferably, the achievement of the effectiveness of the plasma gas can be determined on the basis of the parameters established by the processing means with respect to the values of the environmental parameters, generally temperature and humidity, acquired by the combined detection sensor in the volume of air contained in the confined environment regardless of the number of objects detected therein which could subtract, as a percentage, a partial volume from the total volume of the confined environment C.

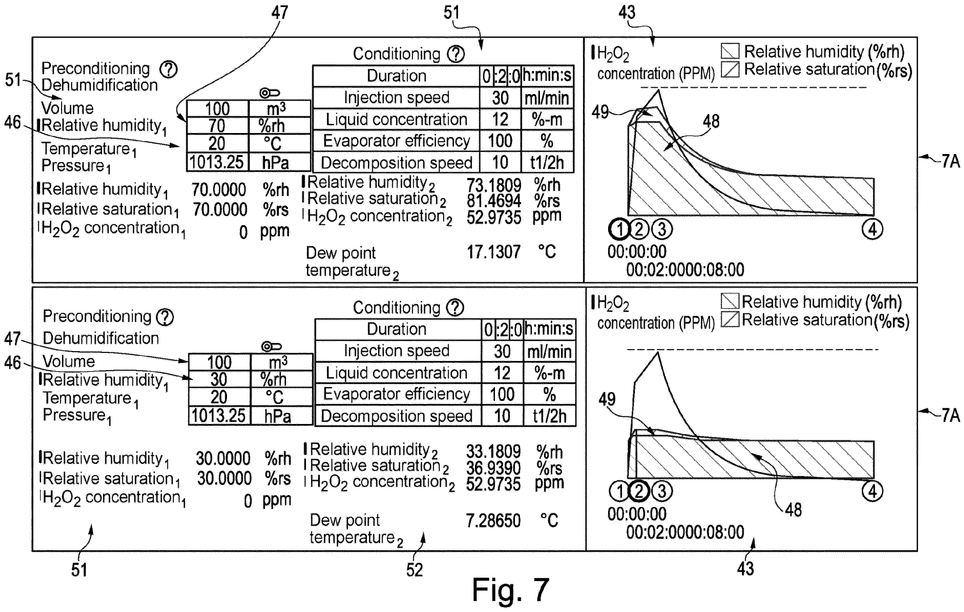

The certification of successful disinfection treatment of the surfaces of the confined environment and, possibly, of the objects contained therein and of the air can also be expressed in graphic form 43 (figure 7, figure 8 and figure 10).

In detail, figure 7 depicts two graphs automatically generated by the processing means 10 (i.e., the configuration software) of the control unit 2. The two graphs depict two sanitizing activities, carried out in different environmental conditions, in particular with reference to the relative humidity parameter detected in the start-up step of carrying out the treatment cycle, humidity which is 70% in one case and 30% in the other.

The graph 43 (figure 7 - Tables 7A, 7B) is automatically generated at each treatment cycle in relation to the microbial and volumetric emergence information preset in the control unit 2 integrated with the temperature 46 and relative humidity 47 information detected in the confined environment C at the same time as the start-up step of the treatment cycle.

In figure 7 the two graphs, which as mentioned above are automatically generated by the system, differ, despite having the same settings (since they are the same environment), in the optimal execution curve because the relative humidity detected in the start-up step for carrying out the cycle is different.

An optimal execution diagram produced automatically by the control and processing means based on the environmental parameters which are detected in the start-up step is illustrated in detail. Said control and processing means elaborate a graph which acts as a "guideline" to establish the operating parameters and the amount of product necessary for carrying out the cycle, on the basis of which they enable the use of the product and start its dispersion in the environment.

In figure 7 the two graphs, as mentioned above, are automatically generated by the system according to an optimal execution diagram based on the environmental parameters which are detected in the start-up step. The two graphs differ despite having the same settings (since they are the same environment), in the optimal execution curve because the relative humidity detected in the start-up step for carrying out the cycle is different.

Figure 10 depicts a graph related to the configuration procedure of an execution cycle guideline, as established within the operating protocol, which is processed in the start-up step of the treatment cycle by means of detection of the environmental microclimatic parameters which define a set of concentration curves 48, 49, 50 relating respectively to the humidity, active substance 58 and plasma gas 42 parameters.

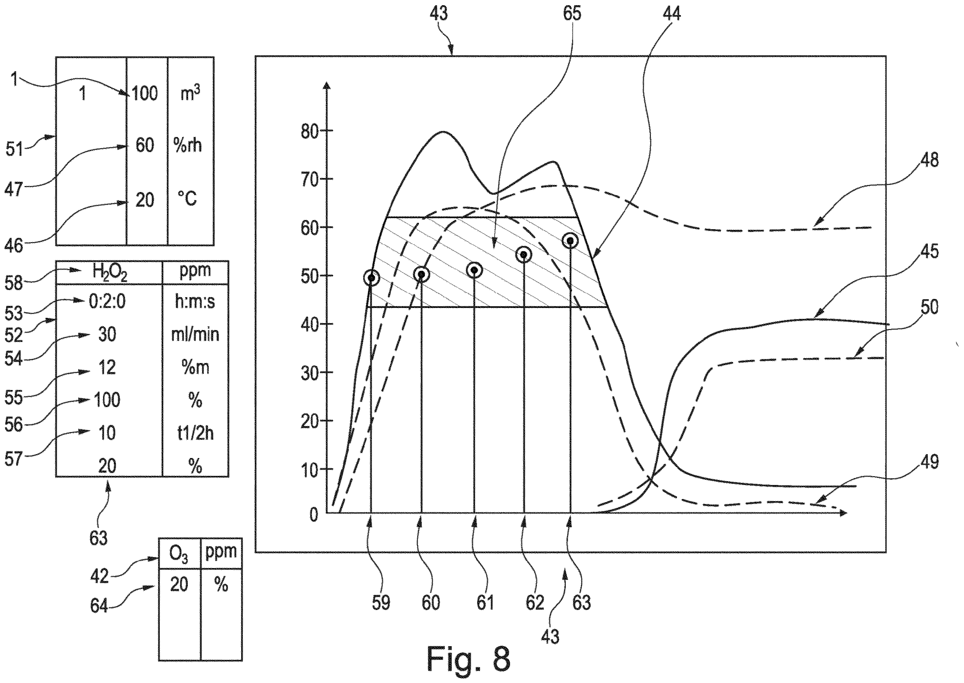

Figure 8 depicts a graph related to the actual execution of the treatment cycle superimposed on graph 10 and on the effective concentration curves 44 of the active substance 58 of the decontaminant 11, of the concentration 45 of the plasma gas 42, of the effective execution temperature 66 and actual execution humidity 67 generated with respect to the detailed information acquired during the operational execution of the procedure, such as: the dispensing speed, the concentration of the liquid product used, the dispensing speed, the times for restoring the minimum safety conditions, the concentration curve of a decontaminant, the concentration curve of a gas, the stages of achieving effectiveness for a single bacterial strain with respect to expected reference values.

The graph in figure 8 shows a first curve 44 and a second curve 45. The curve 44 and curve 45 depict the variation over time, respectively, of a concentration of decontaminant 11 and a concentration of plasma gas 42, both dispersed in the air in the confined environment C during the procedure for carrying out a disinfection activity of the surfaces, objects and air contained in a confined environment C.

The graph 43 (Figure 7, Tables 7A and 7B) shows three lines indicative of a value and an execution time (which can change with respect to the simple variation of one of the parameters detected, for example the temperature 46), according to mathematical calculations, with respect to: a relative humidity curve 48, a relative saturation curve 49 of the active substance 58 and a relative concentration curve 50 of a plasma gas 42 of concentration 50.

In Figure 8 the graph, with respect to the graphs in Figure 7, depicts the actual execution of the cycle. As mentioned above, some parameters during the execution of the treatment could vary further, forcing, for example, the device to be turned back on. The objective is to maintain the parameters indicated in the optimal treatment execution curve for as long as possible (graphs in Figure 7). In practice, if the two graphs 7 and 8, superimposed, were the same, it would mean that we performed a perfect disinfection.

The control unit 2, by means of the control means 9 and processing means 10, calculates the various parameters of the operating protocol (Figure 7 and 8 - table 52) i.e., for example, the type of active substance 58 to be used, its concentration 55 in the liquid vehicle (water), the duration of the treatment 53, the dispensing speed 54, the dimension of the particles to be dispersed in the air 56 and the action and contact time divided by single microorganism, bacteria 59, fungi 60, viruses 61, spores 62 mycobacteria 63 and the information on restoring 57 the minimum safety conditions. Subsequently, if present, it activates the air treatment device 4 which dispenses the plasma gas 42. The air treatment allows to maintain the sanitizing result achieved in compliance with the quality parameters established in the operating protocol of use adopted.

According to a preferred embodiment, in the method of the present invention it is envisaged to verify whether the concentration curve 44 of decontaminant 11 dispensed is consistent with the parameters established by the control unit 2, specifically by the control means 9 and processing means 10 , shown in the graph 43, to certify the replicability of the intervention according to the specifications contained in the EN standards for each individual microorganism of interest.

In the example of figure 8, graph 43 refers to a monitoring step of the disinfection method carried out in a confined environment C of 100 cubic metres in temperature conditions greater than or equal to 20 degrees centigrade and humidity greater than or equal to 60 percent (Table 51). The control unit 2 processes, in the start-up step of the treatment cycle and at the same time as the detection of the environmental microclimatic parameters, an operating protocol regarding an optimal execution path which establishes the coordination (switching on and off) of the devices 3 and 4 and the objectives (contact time and action) to be achieved by means of the use of the active substance 58 and the plasma gas 42. The following are graphically depicted on the graph 43: a relative humidity curve 48, a relative saturation curve of the active substance 49 and a concentration curve 50 of the plasma gas. The expected microbiological values to be achieved are also indicated on the graph 43, in particular with regard to the action on bacteria 59, fungi 60, viruses 61, spores 62, mycobacteria 63.

The intervention certification occurs as a function of the effective monitoring of the active substance 58 and the plasma gas 42, both dispersed in the air in a confined environment C, and detected by means of first sensor means 7 and second sensor means 8, respectively. Such monitoring produces data with which a concentration curve 44 of an active substance 58 of a decontaminant 11 and a curve 45 of plasma gas 42 are graphically generated. Said curves are constructed based on the actual concentration values, expressed in parts per million, and the action and contact times. The chronology of the activities and their conformity with respect to the procedure established in the operating protocol is documented on a summary report of the intervention, processed by the control means 9 and processing means 10 to be transferred to the central server 40 and archived.

## Claims

1. A method for carrying out, tracing, monitoring and controlling an airborne disinfection treatment of surfaces of a confined environment (C) and of at least one target object (A) present therein, said method being implemented through the use of a system of devices (1) comprising at least:
- a sanitizing apparatus (3) of the surfaces provided with at least dispensing means (31) of a liquid decontaminant (11) contained in an interchangeable pack (25), and
- a control unit (2) comprising at least:
a) first sensor means (7) for detecting the concentration of said decontaminant (11) in the confined environment (C),
b) a combined sensor (41), and
c) control means (9) and processing means, connected to said first sensor means (7),
said control unit (2) being configured to contain identification information of the confined environment (C);
wherein said combined sensor (41) is designated to detecting at least one environmental parameter relating to at least one piece of data selected from microclimate data and data concerning the presence of at least one pollutant, and
wherein said decontaminant (11) is released in the form of mist, the dispensing of which is controlled as a function of at least one of the pieces of data detected by said first sensor means (7), said dispensing being conducted according to a predetermined operating protocol for carrying out the treatment,
said method comprising the steps of:
a) associating the control unit (2) with the confined environment (C);
b) arranging said target object (A) in the confined environment (C);
c) arranging said sanitizing apparatus (3) in the confined environment;
d) acquiring information concerning at least said environmental parameter by means of said combined sensor (41);
e) processing, with said control means (9) and processing means (10), said information acquired by means of said combined sensor (41) producing said operating protocol for carrying out the disinfection treatment, the disinfection mode being defined in said protocol;
f) transferring data relating to the disinfection protocol to the sanitizing apparatus (3) to initiate the disinfection treatment of the confined environment (C) and of the at least one object (A);
g) inserting the interchangeable pack (25) into the sanitizing apparatus (3);
h) dispersing into the air said decontaminant (11) according to a concentration and for a dispensing time calculated by the control means (9) and processing means (10), based on the information contained in said operating protocol; and
i) making available certification information, according to the concentration of decontaminant (11) detected, to certify that disinfection of the surfaces of the confined environment and of the at least one object present therein has taken place during the step of dispensing the decontaminant.

2. The method according to claim 1, further comprising the following steps:
α) detecting the concentration of the decontaminant dispersed in the air in the confined environment; and
β) calibrating operation of the misting device modulating the amount and input of decontaminant dispersed in the air inside the confined environment in conformity with the specifications indicated in the operating protocol.

3. The method according to any one of the preceding claims, further comprising enabling the use of the disposable and single dose pack (25) containing the decontaminant (12) inserted into the sanitizing apparatus (3) by means of recognition means (12) of the decontaminant (11), said enabling taking place in step (g) and after insertion of the interchangeable pack (25) into the sanitizing apparatus (3).

4. The method according to any one of the preceding claims, wherein during dispensing of the decontaminant, a concentration curve of decontaminant (11) dispersed in the air in the confined environment (1) is detected by means of said first sensor means (7).

5. The method according to any one of the preceding claims, further comprising the following steps:
δ) arranging an air treatment device, dispenser of cold plasma gas, inside a confined environment;
ε) transferring the operating specifications according to the operating protocol produced during said step (e) and the amount of plasma gas to disperse into the air of the environment to the air treatment device;
η) detecting the concentration of plasma gas dispersed in the air by the air treatment device in the confined environment;
θ) calibrating operation of the air treatment device modulating the amount of plasma gas dispersed in the air inside the confined environment in conformity with the specifications indicated in the operating protocol produced during said step (e); and
κ) providing certification information relating to the concentration of plasma gas detected as a function of the exposure time required to eliminate the bacterial strains established during configuration of the operating protocol;
said step (δ) being conducted before the aforesaid step (e), the steps (ε), (η) and (θ) being carried out, generally, after said step (d), and said step (κ) being carried out, usually but not necessarily, after the step (i).

6. The method according to the preceding claim, wherein the step (δ) is typically conducted before or after any of the steps from (a) to (c).

7. The method according to any one of the preceding claims, wherein said environmental parameters detected in the confined environment (C) are temperature and humidity, said environmental parameters being monitored during dispensing of said decontaminant (11).

8. The method according to any one of the preceding claims, wherein dispensing of the decontaminant (11) is regulated in such a way that the concentration of said decontaminant (11) dispersed in the air in the confined environment (C) does not drop below a predetermined concentration value curve (55) for a predetermined duration (65).

9. The method according to any one of the preceding claims, further comprising a step (γ) of sending information coming from an identification device (5) associated with at least one target object (A) to recognition means (6) comprised in the control unit (2).

10. A system of devices (1) for carrying out, tracing, monitoring and controlling an airborne disinfection treatment of surfaces of a confined environment (C) and of at least one object (A) present therein, said system (1) comprising at least:
- a sanitizing apparatus (3) of the surface provided with at least dispensing means (31) of a liquid decontaminant (11) contained in an interchangeable pack (25), and
- a control unit (2) comprising at least:
a) first sensor means (7) for detecting the concentration of said decontaminant (11) in the confined environment (C),
b) a combined sensor (41), and
c) control means (9) and processing means, connected to said first sensor means (7),
said control unit (2) being configured to contain identification information of the confined environment (C);
wherein said combined sensor (41) is designated to detecting at least one environmental parameter relating to at least one piece of data selected from microclimate data and data concerning the presence of at least one pollutant, and
wherein said decontaminant (11) is released in the form of mist.

11. The system of devices (1) according to claim 10, further comprising an identification device (5) associated with at least one target object and recognition means (6) comprised in the control unit (2).

12. The system of devices (1) according to claim 10 or 11, wherein the control unit (2) further comprises processing means (10), memory means (19), and optionally second sensor means (8) for detecting gases dispersed in the air in the confined environment (C).

13. The system of devices (1) according to any one of the preceding claims from 10 to 12, wherein the sanitizing apparatus (3) further comprises recognition means (12) of a pack (25) containing the decontaminant (11).

14. The system of devices (1) according to claim 12, wherein said identification device (5) of the at least one target object (A) is a sensor device configured to read labels with RFID, QR code, barcode or BLE hardware technology.

15. The system according to any one of the preceding claims from 10 to 15, further comprising at least one combined detection sensor (41) of at least one environmental microclimate parameter in the volume of air contained in the confined environment (C).
